(19) Europäisches Patentamt
European Patent Office
Office européen des brevets

(11) Veröffentlichungsnummer: **0 072 532**
**A1**

# (12) EUROPÄISCHE PATENTANMELDUNG

(21) Anmeldenummer: 82107271.7

(22) Anmeldetag: 11.08.82

(51) Int. Cl.³: C 07 D 235/28
A 61 K 31/415

(30) Priorität: 14.08.81 DE 3132167

(43) Veröffentlichungstag der Anmeldung: 23.02.83 Patentblatt 83/8

(84) Benannte Vertragsstaaten: AT BE CH DE FR GB IT LI LU NL SE

(71) Anmelder: HOECHST AKTIENGESELLSCHAFT
Postfach 80 03 20
D-6230 Frankfurt/Main 80(DE)

(72) Erfinder: Loewe, Heinz, Dr.
Berliner Ring 6
D-6233 Kelkheim (Taunus)(DE)

(72) Erfinder: Düwel, Dieter, Dr.
Frankfurter Strasse 39
D-6238 Hofheim am Taunus(DE)

## (54) 5(6)-Phenylsulfonyloxy-benzimidazolderivate, Verfahren zu ihrer Herstellung, sie enthaltende pharmazeutische Zubereitungen und ihre Verwendung gegen Leberegel.

(57) Es werden neue 5(6)-Phenylsulfonyloxy-benzimidazolderivate der Formel (1)

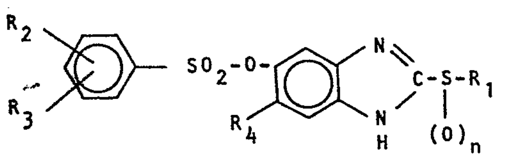

(1)

und ihre Salze mit physiologisch verträglichen Säuren, beschrieben, Verfahren zu ihrer Herstellung, sie enthaltende pharmazeutische Zubereitungen und ihre Wirkung gegen Helminthen, insbesondere gegen Leberegel.

EP 0 072 532 A1

5(6)-Phenylsulfonyloxy-benzimidazolderivate, Verfahren zu ihrer Herstellung, sie enthaltende pharmazeutische Zubereitungen und ihre Verwendung gegen Leberegel

2-Alkylthio-benzimidazolderivate mit Phenoxy- und Phenylthioresten in 5(6)-Stellung sind als Anthelminthica bekannt, ebenso entsprechende 2-Alkylsulfinyl- und 2-Alkylsulfonyl-derivate (DE-OS 28 15 621).

Gegenstand der Erfindung sind anthelminthisch wirksame 2-Alkylthio-, 2-Alkylsulfinyl- und 2-Alkylsulfonylbenzimidazolderivate, die in 5(6)-Stellung durch einen substituierten Phenylsulfonyloxy-rest substituiert sind und durch die Formel (1) wiedergegeben werden,

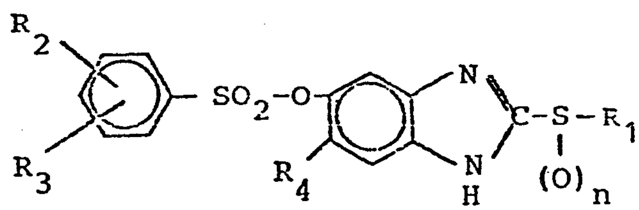

(1)

und ihre Salze mit physiologisch verträglichen Säuren, in welcher

$R_1$ Alkyl mit 1 bis 4 C-Atomen,

$R_2$ und $R_3$ jeweils unabhängig voneinander Wasserstoff, Alkyl mit 1 bis 4 C-Atomen, Alkoxy mit 1 bis 4 C-Atomen, Halogen, Trifluormethyl,

$R_4$ Wasserstoff oder Halogen und

n 0, 1 oder 2 bedeuten.

Hinsichtlich ihrer Wirksamkeit bevorzugt sind solche Verbindungen der Formel I, in welcher

$R_1$ eine Alkylgruppe mit 1 bis 4 C-Atomen,

$R_2$ Halogen wie Fluor, Chlor, Brom, Jod, oder die Trifluormethylgruppe,

$R_3$ Wasserstoff, Halogen wie Fluor, Chlor, Brom, Jod, oder die Trifluormethylgruppe,

0072532



$R_4$ Wasserstoff oder Chlor und
n 0 oder 1 bedeuten.

Besonders bevorzugt sind die Verbindungen der Formel (1a),

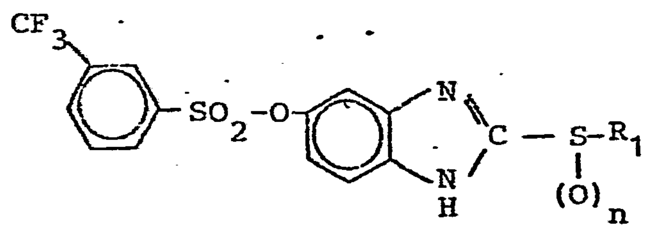

(1a)

in welcher $R_1$ Alkyl mit 1 - 4 C-Atomen, insbesondere Methyl, und n 0 oder 1 bedeuten.

Gegenstand der Erfindung ist ebenso ein Verfahren zur Herstellung der Verbindungen der Formel (1), das dadurch gekennzeichnet ist, daß man eine Verbindung der Formel (2)

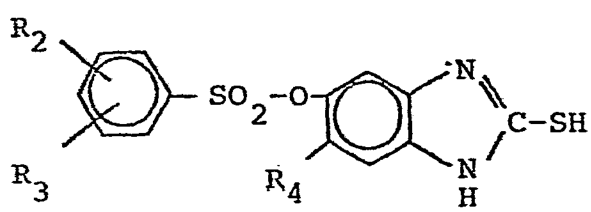

(2)

mit einem Alkylierungsmittel der Formel (3)

$$R_1 - X \qquad (3)$$

umsetzt, wobei $R_1$, $R_2$, $R_3$ und $R_4$ die zu Formel (1) angegebenen Bedeutungen haben und X für Halogen, vorzugsweise Jod oder Brom, einen Mono-Alkylsulfatrest, vorzugsweise den Monomethylsulfatrest, oder

den p-Toluolsulfonylrest steht, und gewünschtenfalls die so erhaltene Verbindung der Formel I, worin n gleich 0 ist, oxydiert.

Die Alkylierung wird bei Temperaturen zwischen 0 und 100°C, vorzugsweise zwischen 20 und 80°C, in Wasser, einem organischen Lösungsmittel oder in Gemischen davon, vorteilhaft in Gegenwart einer Base durchgeführt. Wird eine organische Base im Überschuß verwendet, so kann ein weiteres Lösungsmittel entfallen.

Als organische Lösungsmittel sind beispielsweise Alkohole, Ester, Äther oder Ketone verwendbar. Als Basen kommen beispielsweise Alkali, Alkalikarbonate, -bikarbonate und die entsprechenden Erdalkaliverbindungen, organische Basen wie Pyridin, Collidin, Lutidin oder Picolin oder tertiäre Amine in Betracht.

Die Isolierung der freien Alkylmercapto-benzimidazolderivate der Formel (1) erfolgt zweckmäßig durch Aufnehmen des Reaktionsgemisches der Alkylierung in einem mit Wasser nicht mischbaren inerten Lösungsmittel, z.B. in einem Ester wie Essigsäureäthylester, oder in einem chlorierten Kohlenwasserstoff wie Methylenchlorid, Auswaschen der anorganischen Ionen mit Wasser, Abtrennen der wäßrigen Lösung und Verrühren des eingedampften Rückstandes der organischen Phase mit einem Äther wie Diisopropyläther. Arbeitet man mit Alkyljodid ohne Verwendung einer Base, so fallen die Hydrojodide der Alkylmercaptoverbindungen der Formel 1 direkt an. In anderen Fällen kann man, wenn gewünscht, Salze der Verbindungen der Formel (1) durch Zugabe einer physiologisch verträglichen Säure, vorzugsweise einer Mineralsäure wie Salzsäure, Schwefelsäure oder Phosphorsäure, erhalten.

Die Oxidation der Verbindungen der Formel (1), worin n gleich 0 ist, kann bei Temperaturen zwischen -20 und +50°C, vorzugsweise zwischen 0 und 30°C durchgeführt werden. Als Oxydationsmittel kommen in Betracht vorzugsweise äquivalente Mengen von Hydroperoxyd in einer organischen Säure, vorzugsweise in Eisessig, Persäuren wie z.B. Peressigsäure, Pertrifluoressigsäure oder Metachlorperbenzoesäure sowie Salpetersäure oder Chromsäure in Eisessig, bzw. die Salze dieser Säuren, ferner Permanganate, Hypochlorite, Perchlorate, Perjodate und Stickoxyde. Wenn die Einführung nur eines Sauerstoffatomes pro Molekül beabsichtigt ist, wird bevorzugt n-Chlorperbenzoesäure verwendet. Ist die Einführung von zwei Sauerstoffatomen pro Molekül beabsichtigt, so wird bevorzugt Peressigsäure oder Wasserstoffperoxyd verwendet.

Die vorstehend geschilderten Verfahren sind als Analogieverfahren bekannt nach J.chem.Soc. 1949, 3311 - 3315.

Die zur Herstellung der Verbindungen der Formel (1) verwendeten Ausgangsstoffe der Formel (2) lassen sich aus den nach DE-OS 24 41 201 bekannten o-Phenylendiaminderivaten durch Umsetzung mit Schwefelkohlenstoff erhalten (vgl. DE-OS 28 15 621).

Die erfindungsgemäßen Verbindungen sind Chemotherapeutika zur Bekämpfung von Wurmkrankheiten bei Mensch und Tier. Diese werden von Helminthen verursacht, von Cestoden, Nematoden und Trematoden. Die Verbindungen der Formel (1) wirken z.B. gut gegen Fasciolide. Besonders ausgeprägt ist die Wirksamkeit gegen den großen Leberegel Fasciola hepatica. Die Applikation erfolgt an einem bis fünf aufeinander folgenden Tagen, oral oder auch parenteral, mit 1-50 mg/kg, vorzugsweise 2,5-15 mg/kg Körpergewicht.

Zur Feststellung der Wirkung der erfindungsgemäßen Verbindungen gegen Fasciolide wurden chemotherapeutische Untersuchungen bei ca. 250 g schweren Ratten, bei 2 - 3,5 kg schweren Kaninchen oder an ca. 25 - 30 kg schweren Schaflämmern ausgeführt. Die Versuchstiere wurden mit 15, mit 50 bzw. mit 350 Metazerkarien von Fasciola hepatica artifiziell oral infiziert. Nach Ablauf der Präpatenzperiode (Zeit zwischen Infektion und Geschlechtsreife der Parasiten mit beginnender Ausscheidung von Vermehrungsprodukten) wurde der Parasitenbefall durch Kotuntersuchungen gesichert. Der qualitativ-quantitative Nachweis von Eiern erfolgte nach der Telemann-Anreicherung (Deutsche Medizinische Wochenschrift 34: 1510, 1908).

Unmittelbar nach der Bestimmung der Eizahl pro Gramm Kot wurde die Behandlung der Versuchstiere (im allgemeinen 3 - 4 Tiere pro Wirkstoff, mindestens aber zwei) vorgenommen. Den Tieren wurde oral eine Suspension von unterschiedlicher Wirkstoffmenge in jeweils festgelegten Volumen (Ratte: 0,5 ml/Tier, Kaninchen: 2 ml/Tier, Schaf: 10 ml/Tier) in einer Tylose-Suspension appliziert. Jeweils am 7., 14. und 28. Tag nach der Behandlung wurde wiederum nach der vorstehend angegebenen Methode die Eizahl pro Gramm Kot ermittelt und die prozentuale Abnahme im Vergleich zum Ausgangswert vor der Behandlung und in Vergleich zu unbehandelten Kontrolltieren errechnet.

Mit einigen erfindungsgemäßen Verbindungen wurden außerdem beim Fasciola-Befall noch eine zusätzliche Prüftechnik angewandt: Nach Abschluß der Präpatenzperiode wurde bei Fasciola-positiven Schafen ein künstlicher Gallengang geschaffen, der am Ausgang der Gallenblase ein Sieb enthielt, so daß die nach der Behandlung abgetöteten Leberegel in der Gallenblase aufgefangen werden konnten. Vier bis sieben Tage nach der Behandlung wurden die so operierten und dann behandelten Schafe getötet, die abgetöteten Leberegel in der Gallenblase so wie noch eventuell in der Leber vorhandene Leberegel gezählt und von der gesamten Wurmbürde der Prozentsatz der abgetöteten Parasiten berechnet. Bei ausgewählten Substanzen wurden über die Kotuntersuchung hinaus auch die behandelten Tiere seziert und die Leber auf eine eventuelle Rest-Wurmbürde von Fasciola hepatica untersucht.

Eine einmalige Verabreichung der erfindungsgemäßen Wirkstoffe in den angegebenen Dosierungen führt zu einem völligen Aufhören der Eiausscheidung und einer nahezu 100 %igen Befreiung der Tiere von Fasciola hepatica. Alle Dosierungen wurden von den Tieren ohne Nebenerscheinungen vertragen. Dieses wird durch den Sicherheitsfaktor unterstrichen, der wesentlich günstiger ist als bei den zum Vergleich herangezogenen Wirkstoffen

Clioxanide: 2-Acetoxy-4-chlor-3,5-dijod-benzanilid
Nicolofolan: 5,5'-Dichlor-2,2'-dihydroxy-3,3'-dinitrophenyl.

Der Sicherheitsfaktor gibt den Quotienten aus Dosis tolerate maxima und angewandter Dosis an. Die Ergebnisse dieser Versuche sind in der nachstehenden Tabelle zusammengestellt.

**TABELLE**

Wirkung gegen adulte Leberegel bei peroraler Applikation

| Wirkstoff | Ratte mg/kg | Ratte Wirkung % | Kaninchen mg/kg | Kaninchen Wirkung % | Schaf mg/kg | Schaf Wirkung % | Sicherheitsfaktor |
|---|---|---|---|---|---|---|---|
| Verfahrensprodukt Beispiel 1 | 50<br>75 | 80 %<br>100 | < 50<br>75 | 100 %<br>100 | 7,5<br>10,0 | > 95 %<br>100 | > 100<br>> 100 |
| Clioxanide | 135<br>200 | 0<br>0 | 50<br>75 | 0<br>0 | 3,75<br>7,5 | ca. 50<br>> 90 | 30<br>15 |
| Niclofolan | 4<br>8 | 80<br>90 | 4 | Exitus | 1,5<br>3,0 | < 50<br>> 90 | 8,8<br>4,4 |

Die erfindungsgemäßen Verbindungen können in Form von Flüssigkeiten, Pulvern, Tabletten, Kapseln appliziert werden, in Konzentrationen von 2 - 20 % Wirkstoff. Die Verbindungen werden dazu mit Hilfsstoffen gemischt, beispielsweise mit pharmazeutisch üblichen, flüssigen oder festen Füllstoffen, Lösungsmitteln, Emulgatoren, Gleitstoffen, Geschmackskorrigentien, Farbstoffen und/ oder Puffersubstanzen. Außerdem können die Verbindungen auch in Mischung mit geeigneten flüssigen oder festen Futtermitteln verabreicht werden, in entsprechend geringeren Konzentrationen.

Beispiel 1

Man löst 60 g 5(6)-(3-Trifluormethyl-phenylsulfonyloxy)-2-mercaptobenzimidazol in einem Gemisch aus 500 ml Essigester und 500 ml Methanol unter Rückfluß, fügt langsam eine Lösung von 11 ml Methyljodid in 160 ml Essigester zu, hält danach noch 2 Stunden auf Rückflußtemperatur und dampft das Lösungsmittel anschließend ab. Der Rückstand wird hierauf mit Essigester verrührt und der Rückstand abgesaugt. Nach dem Auswaschen mit Essigester und Trocknen verbleiben 72 g Hydrojodid des 5(6)-(3-Trifluormethyl-phenylsulfonyloxy)-2-methylmercapto-benzimidazol vom F.P. 211°C.

Anstelle des Verrührens in Essigester kann man den Rückstand auch in 500 ml heißem Ethanol lösen und mit 1000 ml 2n HCl versetzen. Nach dem Abkühlen und eintägigem Aufbewahren im Eisschrank erhält man nach dem Absaugen, Waschen mit HCl und Aceton 62 g des Hydrochlorids des 5(6)-(3-Trifluormethylphenylsulfonyloxy)-2-methylmercapto-benzimidazol vom F.P. 226°C (Zers.).

Zur Darstellung des als Ausgangsmaterial benötigten 5-(6)-(3-Trifluormethylphenylsulfonyloxy)-2-mercapto-benzimidazols löst man 90,5 g 3-Trifluormethylbenzolsulfonsäure-3-nitro-4-amino-phenylester (DOS 24 41 201, Beispiel 22) in 1000 ml Methanol, hydriert nach Zusatz von Raney-Nickel bei Normaldruck und Normaltemperatur, wobei der entstandene 3-Trifluormethyl-benzolsulfonsäure-3,4-diamino-phenylester beim Eindampfen des Filtrats vom Nickel als Rohprodukt anfällt und ohne weitere Reinigung direkt weiterverarbeitet wird, indem man ihn in 400 ml Methanol löst, 105 ml Schwefelkohlenstoff zufügt und nun eine Lösung aus 44,3 85 %igem KOH in 190 ml absolutem Ethanol bei Raumtemperatur innerhalb von 1 Stunde zutropft. Man rührt das Gemisch hierauf noch 1 Stunde unter Rückfluß.

Tagsdarauf versetzt man mit 1000 ml Essigsäure, verrührt die Mischung noch eine halbe Stunde und saugt den Rückstand ab. Nach dem Auswaschen mit Wasser und Trocknen verbleiben 79 g 5(6)-(3-Trifluormethyl-phenylsulfonyloxy)-2-mercapto-benzimidazol vom F.P. 222°C.

Beispiel 2

Man suspendiert 18,5 g 5(6)-(3-Trifluormethyl-phenylsulfonyloxy)-2-mercaptobenzimidazol in 50 ml n Natronlauge und fügt 150 ml Methanol zu, wobei eine Lösung entsteht. In diese läßt man bei Raumtemperatur 8,5 g Methyljodid tropfen, wobei die Temperatur um 10° ansteigt. Nach zweistündigem Rühren bei Raumtemperatur nimmt man das Reaktionsgemisch in Essigester auf und schüttelt mit Wasser aus, bis keine Jodid-Ionen mehr nachzuweisen sind. Man trocknet die Essigesterlösung über Natriumsulfat und dampft unter vermindertem Druck das Lösungsmittel ab. Den Rückstand nimmt man in 250 ml heißem 97 %igen Ethanol auf, versetzt mit 500 ml 2 n Salzsäure, saugt nach dem Erkalten ab und wäscht mit HCl und Aceton aus. Nach dem Trocknen verbleiben 17 g 5(6)-(3-Trifluormethyl-phenylsulfonyloxy)-2-methylmercapto-benzimidazol-hydrochlorid vom F.P. 226° (Zers.), das mit dem nach Beispiel 1 gewonnenen Material identisch ist.

Analog Beispiel 1 werden dargestellt jeweils aus dem 5(6)-(3-Trifluormethyl-phenylsulfonyloxy)-2-mercapto-benzimidazol durch Umsetzung mit

| | | |
|---|---|---|
| Bsp. 3 | Ethyljodid das | 5(6)-(3-Trifluormethyl-phenylsulfonyloxy)-2-ethylmercapto-benzimidazol-jodid vom F.P. 186°C |
| 4 | Propyljodid | 5(6)-(3-Trifluormethyl-phenylsulfonyloxy)-2-propylmercapto-benzimidazol-jodid vom F.P. 199°C |

Bsp. 5 Butyljodid das 5(6)-(3-Trifluormethyl-phenylsulfonyloxy)-2-butylmercapto-benzimidazol-jodid vom F.P. 138°C

Analog Beispiel 2 werden dargestellt bei jeweiliger Isolierung der freien Base durch Zugabe von Diisopropyläther zum eingedampften Rückstand der mit Wasser ausgewaschenen Lösung des Reaktionsgemisches in Essigester

Bsp. 6 aus 5 (6)-Phenylsulfonyloxy-2-mercapto-benzimidazol vom F.P. 247°C
das 5 (6)-Phenylsulfonyloxy-2-methylmercaptobenzimidazol vom F.P. 116°C

7 aus 5 (6)-(4-Methyl-phenylsulfonyloxy)-2-mercapto-benzimidazol vom F.P. 228°C
das 5 (6)-(4-Methyl-phenylsulfonyloxy)-2-methylmercapto-benzimidazol vom F.P. 125°C

8 aus 5(6)-(4-Chlor-phenylsulfonyloxy)-2-mercapto-benzimidazol vom F.P. 243°C
das, 5(6)-(4-Chlor-phenylsulfonyloxy)-2-methylmercapto-benzimidazol vom F.P. 130°C

9 aus 5(6)-(3-Methyl-phenylsulfonyloxy)-2-mercapto-benzimidazol vom F.P. 236°C
das 5(6)-(3-Methyl-phenylsulfonyloxy)-2-methylmercapto-benzimidazol vom F.P. 118°C

10 aus 5(6)-(3-Chlor-phenylsulfonyloxy)-2-mercapto-benzimidazol vom F.P. 270°C
das 5(6)-(3-Chlor-phenylsulfonyloxy)-2-methylmercapto-benzimidazol vom F.P. 114°C

Bsp. 11 aus 5(6)-(3-Methoxy-phenylsulfonyloxy)-2-mercapto-benzimidazol vom F.P. 230°C
das 5(6)-(3-Methoxy-phenylsulfonyloxy)-2-methyl-mercapto-benzimidazol vom F.P. 104°C

12 aus 5(6)-(4-Isopropyl-phenylsulfonyloxy)-2-mercapto-benzimidazol vom F.P. 248°C
das 5(6)-(4-Isopropyl-phenylsulfonyloxy)-2-methyl-mercapto-benzimidazol vom F.P. 190°C (Zers.)

Beispiel 13

Man löst 20 g 5(6)-(3-Trifluormethyl-phenylsulfonyloxy)-2-methylmercapto-benzimidazol in 750 ml Chloroform und tropft bei einer Temperatur von 0 - 5°C eine Lösung von 10 g 90 %iger m-Chlorperbenzoesäure in 250 ml Chloroform innerhalb einer halben Stunde unter Rühren dazu. Das Reaktionsgemisch wird noch 3 Stunden bei 0 bis 5°C und dann über Nacht bei Raumtemperatur weitergerührt. Das Filtrat wird zur Entfernung überschüssiger m-Chlorperbenzoesäure mit Bisulfitlösung ausgeschüttelt, sodann mit Wasser gewaschen und über Calciumchlorid getrocknet. Man dampft unter vermindertem Druck ein und kristallisiert den Rückstand, der aus rohem 5(6)-(3-Trifluormethyl-phenylsulfonyloxy)-2-methylsulfinyl-benzimidazol besteht, aus Essigester um.

Beispiel 14

Man löst 20 g 5(6)-(3-Trifluormethyl-phenylsulfonyloxy)-2-methylmercapto-benzimidazol in 150 ml Eisessig und tropft innerhalb einer halben Stunde bei Raumtemperatur eine Lösung von 25 ml 40 %iger Peressigsäure dazu. Man rührt über Nacht bei Raumtemperatur und fällt sodann durch Zugabe von Wasser das entstandene 5(6)-(3-Trifluormethyl-phenylsulfonyloxy)-2-methylsulfonyl-benzimidazol aus.

PATENTANSPRÜCHE:

1. 5(6)-Phenylsulfonyloxy-benzimidazolderivate der Formel (1)

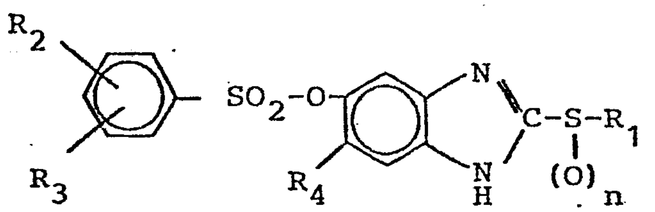

(1)

und ihre Salze mit physiologisch verträglichen Säuren, in welcher
$R_1$ Alkyl mit 1 bis 4 C-Atomen,
$R_2$ und $R_3$ jeweils unabhängig voneinander Wasserstoff, Alkyl mit 1 bis 4 C-Atomen, Alkoxy mit 1 bis 4 C-Atomen, Halogen, Trifluormethyl,
$R_4$ Wasserstoff oder Halogen und
n 0, 1 oder 2 bedeuten.

2. Verfahren zur Herstellung der Verbindungen der Formel (1) in Anspruch 1, dadurch gekennzeichnet, daß man
eine Verbindung der Formel (2)

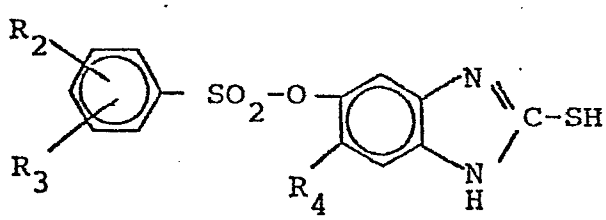

(2)

mit einem Alkylierungsmittel der Formel (3)

$$R_1 - X \qquad (3)$$

umsetzt, wobei $R_1$, $R_2$, $R_3$ und $R_4$ die zu Formel (1) in Anspruch 1 angegebenen Bedeutungen haben und X für Halogen, einen Mono-Alkylsulfatrest, oder den p-Toluolsulfonylrest steht,

und gewünschtenfalls die so erhaltene Verbindung der Formel I, worin n gleich 0 ist, oxydiert, und/oder gegebenenfalls die so erhaltene Verbindung mit einer physiologisch verträglichen Säure versetzt.

3. Arzneimittel, dadurch gekennzeichnet, daß es ein 5(6)-Phenylsulfonyloxy-benzimidazolderivat der Formel (1) in Anspruch 1 enthält oder aus ihm besteht.

4. Verwendung eines 5(6)-Phenylsulfonyloxy-benzimidazolderivates der Formel (1) in Anspruch 1 zur Herstellung eines Arzneimittels gegen Helminthen.

5. Verwendung eines 5(6)-Phenylsulfonyloxy-benzimidazolderivates der Formel (1) in Anspruch 1 zur Bekämpfung von Helminthen in Mensch oder Tier.

6. 5(6)-(3-Trifluormethyl-phenylsulfonyloxy)-2-methylmercapto-benzimidazol.

Patentanspruch für Österreich:

Verfahren zur Herstellung von 5(6)-Phenylsulfonyloxy-benzimidazolderivaten der Formel (1)

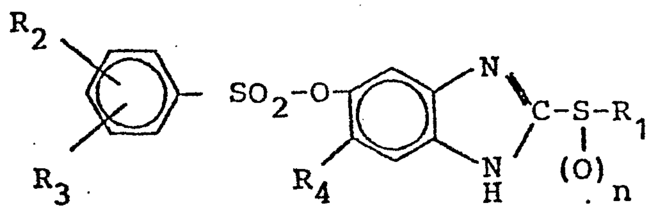

(1)

und ihrer Salze mit physiologisch verträglichen Säuren, in welcher

$R_1$ Alkyl mit 1 bis 4 C-Atomen,

$R_2$ und $R_3$ jeweils unabhängig voneinander Wasserstoff, Alkyl mit 1 bis 4 C-Atomen, Alkoxy mit 1 bis 4 C-Atomen, Halogen, Trifluormethyl,

$R_4$ Wasserstoff oder Halogen und

n 0, 1 oder 2 bedeuten,

dadurch gekennzeichnet, daß man eine Verbindung der Formel (2)

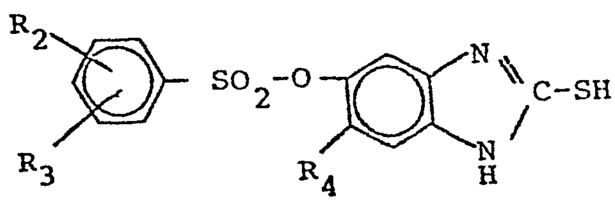

(2)

mit einem Alkylierungsmittel der Formel (3)

$$R_1 - X \qquad (3)$$

umsetzt, wobei $R_1$, $R_2$, $R_3$ und $R_4$ die zu Formel (1) angegebenen Bedeutungen haben und X für Halogen, einen Mono-Alkylsulfatrest, oder den p-Toluolsulfonylrest steht, und gewünschtenfalls die so erhaltene Verbindung der Formel I, worin n gleich 0 ist, oxydiert, und/oder gegebenenfalls die so erhaltene Verbindung mit einer physiologisch verträglichen Säure versetzt.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0072532

Nummer der Anmeldung

EP 82107271.7

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D,A | DE - A1 - 2 815 621 (CIBA)<br>* Seiten10-12 *<br>-- | 1-4 |
| D,A | DE - A1 - 2 441 201 (HOECHST)<br>* Ansprüche 1,3 *<br>-- | 1-4 |
| A | DE - A1 - 2 441 202 (HOECHST)<br>* Ansprüche 1,3 *<br>---- | 1-4 |

KLASSIFIKATION DER ANMELDUNG (Int. Cl.)

C 07 D 235/28
A 61 K 31/415

RECHERCHIERTE SACHGEBIETE (Int. Cl.)

C 07 D 235/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-4,6

Unvollständig recherchierte Patentansprüche: -

Nicht recherchierte Patentansprüche: 5

Grund für die Beschränkung der Recherche: (Artikel 52(4) EPÜ, Verfahren zur therapeutischen Behandlung des menschlichen oder tierischen Körpers)

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| WIEN | 11-11-1982 | BRUS |